(19) Europäisches Patentamt
European Patent Office
Office européen des brevets

(11) Veröffentlichungsnummer: **0 320 864**
**A2**

(12) # EUROPÄISCHE PATENTANMELDUNG

(21) Anmeldenummer: **88120767.4**

(22) Anmeldetag: **13.12.88**

(51) Int. Cl.4: **C07C 69/757 , C07C 62/38**

(30) Priorität: **18.12.87 DE 3743695**

(43) Veröffentlichungstag der Anmeldung:
**21.06.89 Patentblatt 89/25**

(84) Benannte Vertragsstaaten:
**AT BE CH DE ES FR GB GR IT LI LU NL SE**

(71) Anmelder: **SCHERING AKTIENGESELLSCHAFT**
**Berlin und Bergkamen**
**Müllerstrasse 170/178 Postfach 65 03 11**
**D-1000 Berlin 65(DE)**

(72) Erfinder: **Angermann, Alfred, Dr.**
**Stephanstrasse 23**
**D-1000 Berlin 21(DE)**
Erfinder: **Franke, Helga, Dr.**
**Spiessergasse 6 b**
**D-1000 Berlin 27(DE)**
Erfinder: **Johann, Gerhard, Dr.**
**Hermsdorfer Damm 147**
**D-1000 Berlin 28(DE)**

(54) **4-Benzoyl-3-hydroxy-5-oxo-3-cyclohexencarbonsäurederivate, Verfahren zur Herstellung dieser Verbindungen und ihre Verwendung als Mittel mit pflanzenwachstumsregulierender Wirkung.**

(57) Die Erfindung betrifft neue 4-Benzoyl-3-hydroxy-5-oxo-3-cyclohexencarbonsäurederivate der allgemeinen Formel I

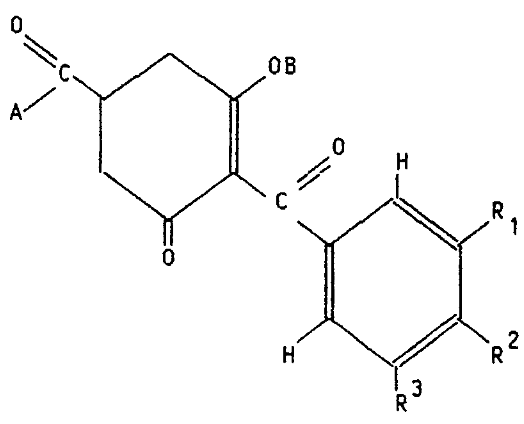

(I) ,

in der A, B, $R^1$, $R^2$ und $R^3$ die in der Beschreibung genannten Bedeutungen haben, Verfahren zur Herstellung dieser Verbindungen und ihre Verwendung als Mittel mit pflanzenwachstumsregulierender Wirkung.

EP 0 320 864 A2

# 4-BENZOYL-3-HYDROXY-5-OXO-3-CYCLOHEXENCARBONSÄUREDERIVATE, VERFAHREN ZUR HERSTELLUNG DIESER VERBINDUNGEN UND IHRE VERWENDUNG ALS MITTEL MIT PFLANZENWACHSTUMSREGULIERENDER WIRKUNG

Die Erfindung betrifft neue 4-Benzoyl-3-hydroxy-5-oxo-3-cyclohexencarbonsäurederivate, Verfahren zur Herstellung dieser Verbindungen und ihre Verwendung als Mittel mit pflanzenwachstumsregulierender Wirkung.

Es ist bereits bekannt, daß gewisse Cyclohexandioncarbonsäurederivate eine herbizide Wirkung besitzen (EP-Anmeldungen 0 137 963 und 0 186 117). Eine pflanzenwachstumsregulierende Wirkung ist bei diesen bekannten Verbindungen bisher nicht beobachtet worden.

Aufgabe der vorliegenden Erfindung ist die Bereitstellung von neuen Verbindungen, die in ihren biologischen Eigenschaften den bisher bekannten Verbindungen überlegen sind.

Diese Aufgabe wird gelöst durch neue 4-Benzoyl-3-hydroxy-5-oxo-3-cyclohexencarbonsäurederivate der allgemeinen Formel I

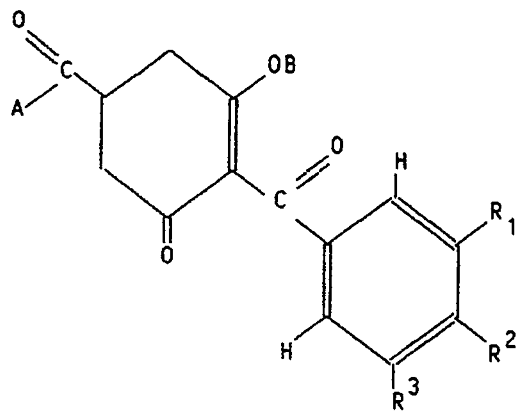

(I) ,

in der

A eine der Gruppen $OR^4$, $NR^5R^6$ oder OM,

B ein Wasserstoffatom oder ein Kation vom Typ M,

M ein Kation aus der Gruppe Lithium, Natrium und Kalium, ein Äquivalent aus der Gruppe Zink, Mangan, Calcium, Magnesium und Barium oder ein Ammoniumion der allgemeinen Formel

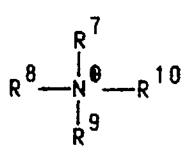

,

$R^1$, $R^2$ und $R^3$, unabhängig voneinander, ein Wasserstoffatom, ein Halogenatom, eine Hydroxy-, Cyano- oder Nitrogruppe, einen $C_1$-$C_6$-Alkyl-, $C_2$-$C_6$-Alkenyl-, $C_2$-$C_6$-Alkinyl-, $C_1$-$C_{10}$-Alkoxy-, Phenyl- oder Benzylrest, einen ein- oder mehrfach, gleich oder verschieden durch Halogen, Hydroxy, Halogen-$C_1$-$C_4$-alkyl, $C_1$-$C_4$-Alkoxy, Halogen-$C_1$-$C_4$-alkoxy, $C_1$-$C_4$-Alkylthio, $C_1$-$C_4$-Alkylsulfinyl oder $C_1$-$C_4$-Alkylsulfonyl substituierten $C_1$-$C_6$-Alkyl-, $C_2$-$C_6$-Alkenyl-, $C_2$-$C_6$-Alkinyl-, $C_1$-$C_{10}$-Alkoxy-, Phenyl- oder Benzylrest, einen $C_1$-$C_{10}$-Alkylthio-, $C_1$-$C_{10}$-Alkylsulfinyl- oder $C_1$-$C_4$-Alkylsulfonylrest oder eine der Gruppen $-(CH_2)_nCOA$, $-(CH_2)_nCOR^4$ oder $-NR^5R^6$, oder

$R^1$ und $R^2$ gemeinsam eine Methylendioxy-, Isopropylidendioxy- oder Ethylidendioxygruppe,

n eine Zahl 0, 1 oder 2,

$R^4$ ein Wasserstoffatom, einen $C_1$-$C_{18}$-Alkyl-, $C_2$-$C_{12}$-Alkenyl- oder $C_2$-$C_{12}$-Alkinylrest, einen durch Halogen oder Cyano substituierten oder durch Sauerstoff oder Schwefel ein- oder mehrfach unterbrochenen $C_1$-$C_{18}$-Alkyl-, $C_2$-$C_{12}$-Alkenyl- oder $C_2$-$C_{12}$-Alkinylrest, einen $C_3$-$C_6$-Cycloalkyl-oder $C_3$-$C_6$-Cycloalkyl-$C_1$-$C_2$-alkylrest, einen durch Halogen oder Cyano substituierten $C_3$-$C_6$-Cycloalkyl- oder $C_3$-$C_6$-Cycloalkyl-$C_1$-$C_2$-

alkylrest, einen Phenyl- oder Benzylrest, einen durch Halogen, Cyano, $C_1$-$C_4$-Alkyl, $C_1$-$C_4$-Alkoxy, $C_1$-$C_4$-Alkylthio, Nitro oder Trifluormethyl ein- oder mehrfach substituierten Phenyl- oder Benzylrest oder einen 5- oder 6-gliedrigen heterocyclischen Rest,

$R^5$ und $R^6$, die gleich oder verschieden sind, ein Wasserstoffatom, einen $C_1$-$C_{18}$-Alkyl-, $C_2$-$C_{12}$-Alkenyl- oder $C_2$-$C_{12}$-Alkinylrest, einen durch Halogen, Hydroxy oder Cyano substituierten oder durch Sauerstoff oder Schwefel ein- oder mehrfach unterbrochenen $C_1$-$C_{18}$-Alkyl-, $C_2$-$C_{12}$-Alkenyl-oder $C_2$-$C_{12}$-Alkinylrest, einen Phenyl- oder Benzylrest, einen ein- oder mehrfach durch $C_1$-$C_6$-Alkyl, Halogen, $C_1$-$C_6$-Alkoxy oder die Nitrogruppe substituierten Phenyl- oder Benzylrest oder

$R^5$ und $R^6$ gemeinsam mit dem benachbarten Stickstoffatom die Morpholino-, Piperidino- oder Pyrrolidinogruppe und

$R^7$, $R^8$, $R^9$ und $R^{10}$, die gleich oder verschieden sind, ein Wasserstoffatom, einen $C_1$-$C_6$-Alkyl-, Phenyl-, Benzyl- oder Phenylethylrest, einen durch Halogen, Hydroxy oder $C_1$-$C_6$-Alkoxy substituierten $C_1$-$C_6$-Alkyl-, Phenyl-, Benzyl- oder Phenylethylrest

bedeuten.

Die Bezeichnung "Halogen" umfaßt Fluor, Chlor, Brom und Jod. Unter den Begriffen "Alkyl", "Alkenyl" und "Alkinyl" sind sowohl geradkettige als auch verzweigte Kohlenwasserstoffreste zu verstehen. Beispiele für fünf- und sechsgliedrige heterocyclische Reste sind Furan, Pyran, Pyrrol, Pyrrolidin, Pyridin, Piperidin, Pyrazol, Pyrazolidin, Pyrimidin, Oxazolidin, Oxazol, Morpholin, Thiazol, Thiazolidin und Thiophen.

Die erfindungsgemäßen Verbindungen können auch als Tautomere auftreten. Für 4-Benzoyl-3-hydroxy-5-oxo-3-cyclohexencarbonsäurederivate der allgemeinen Formel I beispielsweise lassen sich auch die 3,5-Dioxostrukturen der allgemeinen Formeln I′ und I″

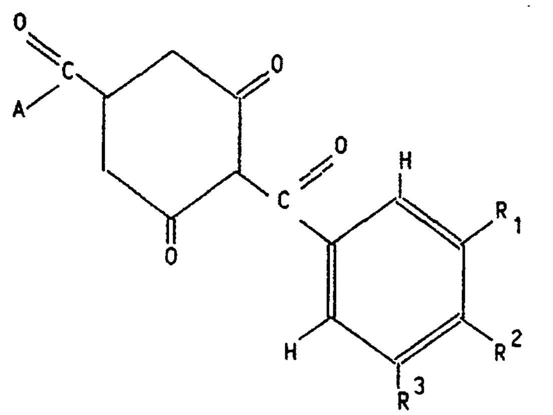

(I′)

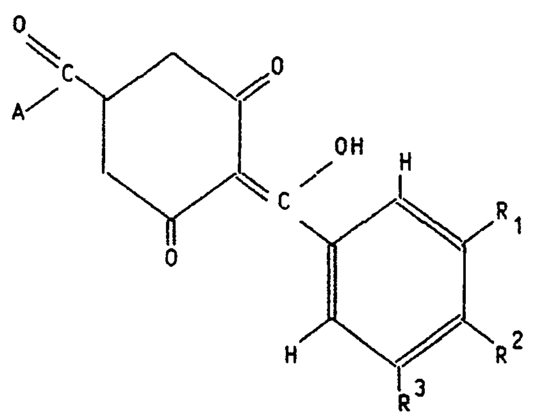

(I″)

formulieren, in denen A, $R^1$, $R^2$ und $R^3$ die unter der allgemeinen Formel I genannten Bedeutungen haben. Diese Strukturen werden ebenfalls durch die vorliegende Erfindung umfaßt, aus Gründen der Einfachheit wird aber jeweils nur die Struktur der allgemeinen Formel I angegeben.

Die erfindungsgemäßen Verbindungen der allgemeinen Formel I lassen sich zum Beispiel herstellen, indem man

A) eine Verbindung der allgemeinen Formel II

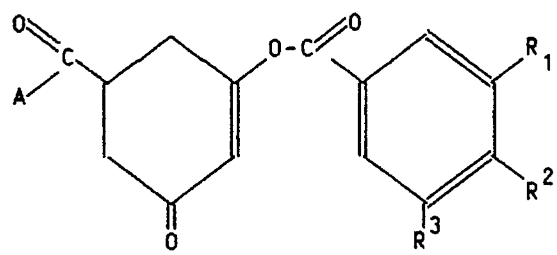

(II) ,

in der A, $R^1$, $R^2$ und $R^3$ die unter der allgemeinen Formel I genannten Bedeutungen haben, einer katalytisch geförderten Umlagerungsreaktion unterwirft, oder

B) eine Verbindung der allgemeinen Formel III

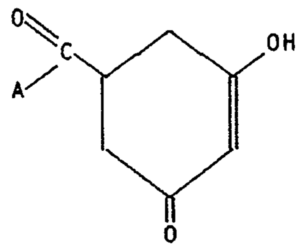

(III) ,

in der A die unter der allgemeinen Formel I genannte Bedeutung hat, in Gegenwart einer Lewis-Säure und einer geeigneten Base mit einem Benzoylcyanid der allgemeinen Formel IV umsetzt

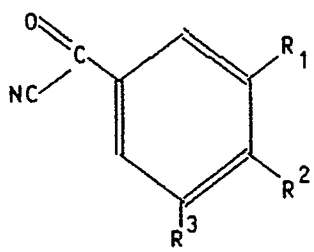

(IV) ,

in der $R'$, $R^2$ und $R^3$ die unter der allgemeinen Formel I genannten Bedeutungen haben, oder

C) eine Verbindung der allgemeinen Formel I a

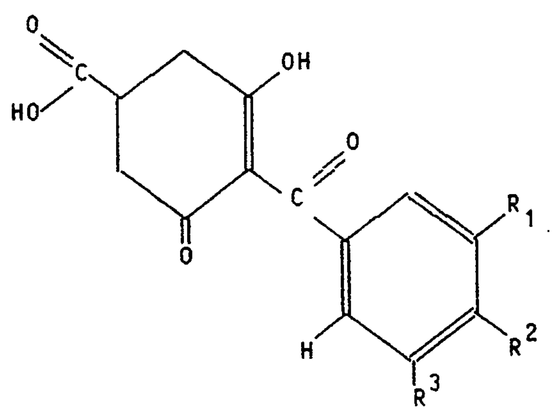

(I a) ,

in der $R^1$, $R^2$ und $R^3$ die unter der allgemeinen Formel I genannten Bedeutungen haben, mit Alkoholen oder Aminen der allgemeinen Formeln V oder VI

$R^4OH$ (V) $HNR^5R^6$ (VI) ,

in denen $R^4$, $R^5$ und $R^6$ die unter der allgemeinen Formel I genannten Bedeutungen haben, zur Reaktion bringt.

Als Katalysatoren für die Umlagerungsreaktion A) eignen sich Pyridinderivate, wie zum Beispiel 4-(N,N-Dialkylamino)-pyridine, vorzugsweise 4-(N,N-Dimethylamino)-pyridin, N-Alkylimidazole, wie zum Beispiel N-Methylimidazol, Metallcyanide, wie zum Beispiel Kupfer-I-cyanid, Natriumcyanid, Kaliumcyanid und Zinkcyanid, 2-Hydroxyisobutansäurenitril oder Lewis-Säuren, wie zum Beispiel Zinkchlorid oder Aluminiumchlorid.

Die Umlagerungsreaktion A) wird vorzugsweise in Anwesenheit eines inerten Lösungsmittels durchgeführt. Als besonders geeignet haben sich hierfür aromatische Kohlenwasserstoffe, wie Benzol, Toluol und Xylol, Halogenkohlenwasserstoffe, wie zum Beispiel Tetrachlorkohlenstoff, Trichlormethan und Dichlormethan, Acetonitril oder organische Basen, wie Triethylamin und Pyridin, erwiesen.

Die Ausgangsstoffe der allgemeinen Formel II erhält man durch Acylierung eines Cyclohexencarbonsäurederivates der allgemeinen Formel III

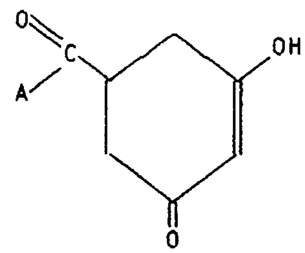

(III) ,

in der A die unter der allgemeinen Formel I genannte Bedeutung hat, mit einem Benzoylhalogenid der allgemeinen Formel VII

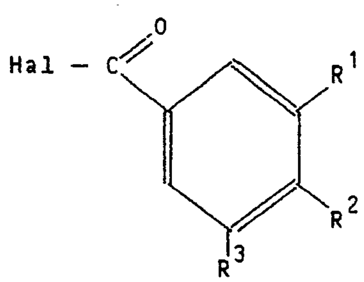

(VII) ,

in der $R^1$, $R^2$ und $R^3$ die unter der allgemeinen Formel I genannten Bedeutungen haben und Hal für ein Halogenatom, vorzugsweise für ein Chlor- oder Bromatom, steht.

Diese Reaktion wird gegebenenfalls in Anwesenheit eines inerten Lösungsmittels und eines säurebindenden Mittels durchgeführt. Als solche säurebindenden Mittel kommen verschiedene organische und anorganische Basen in Betracht, als besonders geeignet haben sich jedoch Triethylamin und Pyridin erwiesen.

Die Herstellungsvariante B) stellt ein "Ein-Topf-Verfahren" dar. Die Reaktion wird praktischerweise so durchgeführt, daß das Benzoylcyanid der allgemeinen Formel IV, die Verbindung der allgemeinen Formel II und die Lewis-Säure bei Raumtemperatur in einem inerten Lösungsmittel zusammengegeben werden und zu dieser Mischung bei Raumtemperatur oder darunter eine Base getropft wird.

Als Lösungsmittel für diese Umsetzung eignen sich die bei Herstellungsvariante A) aufgeführten.

Unter den Lewis-Säuren sind Zink- beziehungsweise Aluminiumchlorid und -bromid bevorzugt. Geeignete Basen sind tertiäre Amine, wie zum Beispiel Triethylamin oder Pyridin.

Bei Herstellungsvariante C) können Standardveresterungs- beziehungsweise Amidierungsmethoden zum Einsatz kommen.

Als besonders vorteilhaft hat sich in diesem Fall die azeotrope Veresterung in Benzol oder Toluol in Gegenwart von p-Toluolsulfonsäure oder Phosphorsäure erwiesen. Gut geeignet sind aber auch diejenigen Verfahren, bei denen eine Wasserabspaltung beispielsweise durch Umsetzung der Komponenten mit Thionylchlorid, Oxalylchlorid, Dicyclohexylcarbodiimid oder dem System Triphenylphosphan/Azodicarbonsäuredialkylester erreicht wird.

Bei den Herstellungsvarianten A), B) und C) können - neben den bereits erwähnten - alle solchen Lösungs- beziehungsweise Verdünnungsmittel zum Einsatz kommen, die gegenüber den Reaktanden inert sind. Beispiele für solche Lösungsmittel beziehungsweise Verdünnungsmittel sind aliphatische, alicyclische

und aromatische Kohlenwasserstoffe, die jeweils gegebenenfalls chloriert sein können, wie zum Beispiel Hexan, Cyclohexan, Petrolether, Ligroin, Benzol, Toluol, Xylol, Methylenchlorid, Chloroform, Kohlenstofftetrachlorid, Ethylenchlorid, Trichlorethylen und Chlorbenzol, Ether, wie zum Beispiel Diethylether, Methylethylether, Methyl-t-butylether, Diisopropylether, Dibutylether, Dioxan und Tetrahydrofuran, Ketone, wie zum Beispiel Aceton, Methylethylketon, Methylisopropylketon und Methylisobutylketon, Nitrile, wie zum Beispiel Acetonitril und Propionitril, Alkohole, wie zum Beispiel Methanol, Ethanol, Isopropanol, Butanol und Ethylenglycol, Ester, wie zum Beispiel Ethylacetat und Amylacetat, Säureamide, wie zum Beispiel Dimethylformamid und Dimethylacetamid, Sulfoxide, wie zum Beispiel Dimethylsulfoxid, und Sulfone, wie zum Beispiel Sulfolan, sowie Basen, wie zum Beispiel Pyridin.

Die Reaktionsvarianten A), B) und C) werden vorzugsweise unter dem Druck der Umgebung durchgeführt, wenngleich sie auch bei erhöhtem oder vermindertem Druck durchgeführt werden könnten. Sie können ferner innerhalb eines breiten Temperaturbereichs durchgeführt werden. Im allgemeinen werden sie bei einer Temperatur zwischen -20 °C und dem Siedepunkt des Reaktionsgemisches, vorzugsweise bei 0 °C bis 150 °C, durchgeführt.

Bei den Herstellungsmethoden A), B) und C) kann die Gegenwart zusätzlicher Reaktionskatalysatoren von Vorteil sein. Als solche Katalysatoren sind Kaliumjodid und Oniumverbindungen, wie quarternäre Ammonium-, Phosphonium-, Arsoniumverbindungen und Sulfoniumverbindungen, geeignet. Ebenfalls geeignet sind Polyglycolether, insbesondere cyclische, wie zum Beispiel 18-Krone-6, und tertiäre Amine, wie zum Beispiel Tributylamin. Bevorzugt sind quarternäre Ammoniumverbindungen, wie zum Beispiel Benzyltriethylammoniumbromid und Tetrabutylammoniumbromid.

Die nach den oben genannten Verfahrensvarianten hergestellten erfindungsgemäßen Verbindungen können nach den üblichen Methoden aus dem Reaktionsgemisch isoliert werden, beispielsweise durch Abdestillieren des eingesetzten Lösungsmittels bei normalem oder vermindertem Druck, durch Ausfällen mit Wasser oder durch Extraktion. Ein erhöhter Reinheitsgrad kann in der Regel durch säulenchromatographische Aufreinigung sowie durch fraktionierte Destillation oder Kristallisation erhalten werden.

Die erfindungsgemäßen Verbindungen stellen in der Regel fast farb- und geruchlose Flüssigkeiten oder Kristalle dar, die bedingt löslich in Wasser, aliphatischen Kohlenwasserstoffen, wie Petrolether, Hexan, Pentan und Cyclohexan, gut löslich in halogenierten Kohlenwasserstoffen, wie Chloroform, Methylenchlorid und Tetrachlorkohlenstoff, aromatischen Kohlenwasserstoffen, wie Benzol, Toluol und Xylol, Ethern, wie Diethylether, Tetrahydrofuran und Dioxan, Carbonsäurenitrilen, wie Acetonitril, Alkoholen, wie Methanol und Ethanol, Carbonsäureamiden, wie Dimethylformamid, Sulfoxiden, wie Dimethylsulfoxid, und in Basen, wie zum Beispiel Pyridin, sind. Aufgrund ihres sauren Charakters lösen sich die erfindungsgemäßen Verbindungen auch in wäßrigen Carbonat-, Hydrogencarbonat- und Hydroxidlösungen.

Die erfindungsgemäßen Verbindungen verursachen sowohl qualitative wie quantitative Veränderungen von Pflanzen als auch Veränderungen im Metabolismus der Pflanzen und sind daher in die Klasse der Pflanzenwachstumsregulatoren einzustufen, die sich durch folgende Anwendungsmöglichkeiten auszeichnen:

- Hemmung des vegetativen Wachstums bei holzigen und krautigen Pflanzen zum Beispiel an Straßenrändern, Gleisanlagen und anderen, um ein zu üppiges Wachstum zu unterbinden. Wuchshemmung beim Getreide, um das Lagern oder Umknicken zu unterbinden, bei Baumwolle zur Ertragserhöhung.
- Beeinflussung der Verzweigung von vegetativen und generativen Organen bei Zier- und Kulturpflanzen zur Vermehrung des Blütenansatzes oder bei Tabak und Tomate zur Hemmung von Seitentrieben.
- Verbesserung der Fruchtqualität, zum Beispiel eine Zuckergehaltssteigerung beim Zuckerrohr, bei Zuckerrüben oder bei Obst und eine gleichmäßigere Reife des Erntegutes, die zu höheren Erträgen führt.
- Erhöhung der Widerstandskraft gegen Streß, so zum Beispiel gegen klimatische Einflüsse, wie Kälte und Trockenheit, aber auch gegen phytotoxische Einflüsse von Chemikalien.
- Beeinflussung des Latexflusses bei Gummipflanzen.
- Ausbildung parthenokarper Früchte, Pollensterilität und Geschlechtsbeeinflussung sind ebenfalls Anwendungsmöglichkeiten.
- Kontrolle der Keimung von Samen oder des Austriebs von Knospen.
- Entlaubung oder Beeinflussung des Fruchtfalles zur Ernteerleichterung.

Die erfindungsgemäßen Verbindungen eignen sich insbesondere zur Hemmung des vegetativen Wachstums bei verschiedenen Pflanzenarten. Eine solche Wuchshemmung wird bisher in der Praxis mit Substanzen verursacht, die entweder nicht konsistent wirken oder in ihrer Wirkung schächer sind als die erfindungsgemäßen Verbindungen.

Dabei handelt es sich zum Beispiel um Stoffe für verschiedene Getreidearten, die eingesetzt werden, um eine Halmverkürzung zu erreichen und der Pflanze eine größere Standfestigkeit zu verleihen. Darüber hinaus können mit einer derartigen Wirkung Pflanzen im Wuchs gehemmt werden, deren Wachstum an

bestimmten Standorten nicht erwünscht ist, so zum Beispiel an Straßenrändern, an Gleisanlagen oder auf Flugplätzen. Auch Grasarten im Rasen können beeinflußt werden, so daß seltener geschnitten werden muß. Wuchshemmende Effekte werden mit solchen Substanzen im allgemeinen auch bei Kulturpflanzen erzielt, die sich durch den Austrieb ruhender Knospen zu intensiv verzweigen, wie zum Beispiel Tabak.

Bei Baumwolle können Wuchshemmungen zu einem gleichmäßigen Ausreifen der Kapseln und dadurch zu höheren Erträgen führen.

Bei Obstbäumen führt bei gezielte Beeinflussung des vegetativen Wachstums zu Ertragssteigerungen und arbeitswirtschaftlichen Vorteilen.

Bei Zierpflanzen erreicht man durch eine Wuchsreduktion oft, daß mehr Pflanzen pro Stellfläche angezogen werden können.

Die erfindungsgemäßen Verbindungen entfalten ihre Wirkung sowohl bei Vorals auch bei Nachauflaufbehandlung. Die Aufwandmengen betragen je nach Anwendungsziel 0,001 bis 5 kg/ha, gegebenenfalls können auch höhere Aufwandmengen eingesetzt werden.

Die Anwendungszeit richtet sich nach dem Anwendungziel und den klimatischen Bedingungen.

Zweckmäßig werden die gekennzeichneten Wirkstoffe oder deren Mischungen in Form von Zubereitungen wie Pulvern, Streumitteln, Granulaten, Lösungen, Emulsionen oder Suspensionen, unter Zusatz von flüssigen und/oder festen Trägerstoffen beziehungsweise Verdünnungsmitteln und gegebenenfalls Haft-, Netz-, Emulgier- und/oder Dispergierhilfsmitteln angewandt.

Geeignete flüssige Trägerstoffe sind zum Beispiel aliphatische und aromatische Kohlenwasserstoffe wie Benzol, Toluol, Xylol, Cyclohexanon, Isophoron, Dimethylsulfoxid, Dimethylformamid, weiterhin Mineralölfraktionen und Pflanzenöle.

Als feste Trägerstoffe eignen sich Mineralien, zum Beispiel Bentonit, Silicagel, Talkum, Kaolin, Attapulgit, Kalkstein, und pflanzliche Produkte, zum Beispiel Mehle.

An oberflächenaktiven Stoffen sind zu nennen zum Beispiel Calciumligninsulfonat, Polyethylenalkylphenylether, Naphthalinsulfonsäuren und deren Salze, Phenolsulfonsäuren und deren Salze, Formaldehydkondensate, Fettalkoholsulfate sowie substituierte Benzolsulfonsäuren und deren Salze.

Der Anteil des beziehungsweise der Wirkstoffe(s) in den verschiedenen Zubereitungen kann in weiten Grenzen variieren. Beispielsweise enthalten die Mittel etwa 10 bis 90 Gewichtsprozent Wirkstoff, etwa 90 bis 10 Gewichtsprozent flüssige oder feste Trägerstoffe sowie gegebenenfalls bis zu 20 Gewichtsprozent oberflächenaktive Stoffe.

Die Ausbringung der Mittel kann in üblicher Weise erfolgen, zum Beispiel mit Wasser als Träger in Spritzbrühmengen etwa 100 bis 1000 Liter/ha. Eine Anwendung der Mittel im sogenannten Low-Volume und Ultra-Low-Volume-Verfahren ist ebenso möglich wie ihre Applikation in Form von sogenannten Mikrogranulaten.

Die Herstellung dieser Zubereitungen kann in an sich bekannter Art und Weise, zum Beispiel durch Mahl- oder Mischverfahren, durchgeführt werden. Gewünschtenfalls können Zubereitungen der Einzelkomponenten auch erst kurz vor ihrer Verwendung gemischt werden, wie es zum Beispiel im sogenannten Tankmixverfahren in der Praxis durchgeführt wird.

Zur Herstellung der verschiedenen Zubereitungen werden zum Beispiel die folgenden Bestandteile eingesetzt:

| **A) Spritzpulver** | | |
|---|---|---|
| 1.) | 20 Gewichtsprozent | Wirkstoff |
| | 68 Gewichtsprozent | Kaolin |
| | 10 Gewichtsprozent | Calciumsalz der Ligninsulfonsäure |
| | 2 Gewichtsprozent | Dialkylnaphthalinsulfonat |
| 2.) | 40 Gewichtsprozent | Wirkstoff |
| | 25 Gewichtsprozent | Kaolin |
| | 25 Gewichtsprozent | kolloidale Kieselsäure |
| | 8 Gewichtsprozent | Calciumsalz der Ligninsulfonsäure |
| | 2 Gewichtsprozent | Natriumsalz des N-Methyl-N-oleyl-taurins |

| B) Wasserlösliches Pulver | |
|---|---|
| 10 Gewichtsprozent | Wirkstoff |
| 75 Gewichtsprozent | Harnstoff |
| 9 Gewichtsprozent | Mischpolymerisationsprodukt aus Ethylenoxid und Propylenoxid |
| 1 Gewichtsprozent | Dialkylnaphthalinsulfonat |
| 5 Gewichtsprozent | Natriumsalz der Ethylendiamintetraessigsäure |

| C) Emulsionskonzentrat | |
|---|---|
| 20 Gewichtsprozent | Wirkstoff |
| 75 Gewichtsprozent | Isophoron |
| 2 Gewichtsprozent | ethoxyliertes Rizinusöl |
| 3 Gewichtsprozent | Calciumsalz der Dodecylphenylsulfonsäure |

Die folgenden Beispiele erläutern die Herstellung der erfindungsgemäßen Verbindungen:

**Beispiel 1**

4-Benzoyl-3-hydroxy-5-oxo-3-cyclohexencarbonsäureethylester

Zu 10,00 g (54,3 mmol) 3,5-Dioxocyclohexancarbonsäureethylester, gelöst in 100 ml Dichlormethan und 15 ml Triethylamin, werden bei 10 °C langsam 7,75 g (55,1 mmol) Benzoylchlorid getropft. Man rührt noch 3 Stunden bei Raumtemperatur nach, gießt auf Eis, trennt ab, schüttelt die organische Phase mit 1 N Salzsäure, gesättigter Bicarbonatlösung und Wasser, trocknet (Magnesiumsulfat) und engt ein. Der so erhaltene 3-Benzoyloxy-5-oxo-3-cyclohexencarbonsäureethylester wird in 75 ml Acetonitril gelöst und nach Zugabe von 15 ml Triethylamin und 1,5 ml 2-Hydroxyisobutansäurenitril 24 Stunden bei Raumtemperatur gerührt. Man nimmt in 100 ml Diethylether auf, extrahiert mit 100 ml 2 N Salzsäure und anschließend zweimal mit je 50 ml 5%iger Kaliumcarbonatlösung. Nach Überschichten der wäßrigen Phase mit 100 ml Diethylether wird mit 4 N Salzsäure bis pH 3 angesäuert, abgetrennt, mit Wasser gewaschen, getrocknet (Magnesiumsulfat) und einrotiert. Zur Reinigung wird aus wenig Hexan umkristallisiert.

| Ausbeute: 8,8 g = 56,3 % der Theorie<br>Fp.: 58-60 °C | | | | |
|---|---|---|---|---|
| Elementaranalyse: | | | | |
| Berechnet: | C | 66,66 % | H | 5,59 % |
| Gefunden: | C | 66,64 % | H | 5,34 % |

**Beispiel 2**

4-(3,4-Dichlorbenzoyl)-3-hydroxy-5-oxo-3-cyclohexencarbonsäureethylester

In eine Lösung von 8,00 g (43,4 mmol) 3,5-Dioxocyclohexancarbonsäureethylester und 8,90 g (44,5 mmol) 3,4-Dichlorbenzoylcyanid in 150 ml Dichlormethan werden insgesamt 6,10 g (4,48 mmol) wasserfreies Zinkchlorid eingetragen. Zu dieser Mischung tropft man bei 0 °C innerhalb von 30 Minuten 4,80 g (47,4 mmol) Triethylamin, rührt anschließend noch 14 Stunden bei Raumtemperatur nach und arbeitet wie bei Beispiel 1 auf. Säulenchromatographische Reinigung (Hexan/Essigester, v/v 80:20) an Kieselgel liefert das gewünschte Produkt.

| | | | | | | |
|---|---|---|---|---|---|---|
| Ausbeute: 8,2 g = 52,8 % der Theorie<br>Fp.: 73-74 °C | | | | | | |
| Elementaranalyse: | | | | | | |
| Berechnet: | C | 53,80 % | H | 3,95 % | Cl | 19,85 % |
| Gefunden: | C | 54,02 % | H | 3,91 % | Cl | 20,13 % |

Analog zu den Beispielen 1 und 2 lassen sich auch die folgenden erfindungsgemäßen Verbindungen herstellen:

| Beispiel | Name der Verbindung | Physikalische Konstante Fp. ($^oC$) / $n_D^{20}$ |
|---|---|---|
| 3 | 4-(3-Methylbenzoyl)-3-hydroxy-5-oxo-3-cyclohexencarbonsäure-ethylester | 38 |
| 4 | 4-(4-Methylbenzoyl)-3-hydroxy-5-oxo-3-cyclohexencarbonsäure-ethylester | 1,5742 |
| 5 | 4-(4-n-Propylbenzoyl)-3-hydroxy-5-oxo-3-cyclohexencarbonsäure-ethylester | 60 |
| 6 | 4-(4-n-Butylbenzoyl)-3-hydroxy-5-oxo-3-cyclohexencarbonsäure-ethylester | 44 |
| 7 | 4-(4-t-Butylbenzoyl)-3-hydroxy-5-oxo-3-cyclohexencarbonsäure-ethylester | 1,5604 |
| 8 | 4-(3-Chlorbenzoyl)-3-hydroxy-5-oxo-3-cyclohexencarbonsäure-ethylester | 1,5758 |
| 9 | 4-(4-Chlorbenzoyl)-3-hydroxy-5-oxo-3-cyclohexencarbonsäure-ethylester | 53 |
| 10 | 4-(3-Fluorbenzoyl)-3-hydroxy-5-oxo-3-cyclohexencarbonsäure-ethylester | 1,5586 |

| Beispiel | Name der Verbindung | Physikalische Konstante Fp.(°C) / $n_D^{20}$ | |
|---|---|---|---|
| 11 | 4-(4-Brombenzoyl)-3-hydroxy-5-oxo-3-cyclohexencarbonsäure-ethylester | 83,5-84 | |
| 12 | 4-(3-Nitrobenzoyl)-3-hydroxy-5-oxo-3-cyclohexencarbonsäure-ethylester | | 1,5778 |
| 13 | 4-(3-Methoxybenzoyl)-3-hydroxy-5-oxo-3-cyclohexencarbonsäure-ethylester | 54 | |
| 14 | 4-(4-Methoxybenzoyl)-3-hydroxy-5-oxo-3-cyclohexencarbonsäure-ethylester | 101-102 | |
| 15 | 4-(4-n-Butyloxybenzoyl)-3-hydroxy-5-oxo-3-cyclohexencarbonsäure-ethylester | 49 | |
| 16 | 4-(3,4-Dimethylbenzoyl)-3-hydroxy-5-oxo-3-cyclohexencarbonsäure-ethylester | 37 | |
| 17 | 4-(3,5-Dimethylbenzoyl)-3-hydroxy-5-oxo-3-cyclohexencarbonsäure-ethylester | | 1,5663 |
| 18 | 4-(3,5-Dichlorbenzoyl)-3-hydroxy-5-oxo-3-cyclohexencarbonsäure-ethylester | 34 | |

| Beispiel | Name der Verbindung | Physikalische Konstante Fp.($^{o}$C) / $n_D^{20}$ |
|---|---|---|
| 19 | 4-(3-Trifluormethylbenzoyl)-3-hydroxy-5-oxo-3-cyclohexencarbonsäureethylester | 45-46 |
| 20 | 4-(3-Chlorbenzoyl)-3-hydroxy-5-oxo-3-cyclohexencarbonsäure-Monohydrat | 84 |
| 21 | 4-(4-Chlorbenzoyl)-3-hydroxy-5-oxo-3-cyclohexencarbonsäure | 181 |
| 22 | 4-(3,4-Dichlorbenzoyl)-3-hydroxy-5-oxo-3-cyclohexencarbonsäure-Monohydrat | 111-127 |
| 23 | 4-(3-Chlorbenzoyl)-3-hydroxy-5-3-cyclohexencarbonsäureethylester - Natriumsalz | 277 |
| 24 | 4-(4-Chlorbenzoyl)-3-hydroxy-5-oxo-3-cyclohexencarbonsäureethylester - Natriumsalz | 303 |
| 25 | 4-(3,4-Dichlorbenzoyl)-3-hydroxy-5-oxo-3-cyclohexencarbonsäureethylester - Natriumsalz | |
| 26 | 4-(3-Methoxybenzoyl)-3-hydroxy-5-oxo-3-cyclohexencarbonsäureethylester - Natriumsalz | 197 |

| Beispiel | Name der Verbindung | Physikalische Konstante Fp.($^{o}$C) / $n_D^{20}$ |
|---|---|---|
| 27 | 4-(3-Fluorbenzoyl)-3-hydroxy-5-oxo-3-cyclohexencarbonsäureethylester - Natriumsalz | 268 |
| 28 | 4-(4-Fluorbenzoyl)-3-hydroxy-5-oxo-3-cyclohexencarbonsäureethylester - Natriumsalz | 281 |
| 29 | 4-Benzoyl-3-hydroxy-5-oxo-3-cyclohexencarbonsäureethylester-Natriumsalz | 252-254 |
| 30 | 4-(4-Ethylbenzoyl)-3-hydroxy-5-oxo-3-cyclohexencarbonsäureethylester | 56 |
| 31 | 3-Hydroxy-4-(4-nitrobenzoyl)-5-oxo-3-cyclohexencarbonsäureethylester | 51-53 |
| 32 | 4-(3,5-Dichlorbenzoyl)-3-hydroxy-5-oxo-3-cyclohexencarbonsäure | 172-173 |
| 33 | 4-(4-Chlorbenzoyl)-3-hydroxy-5-oxo-3-cyclohexencarbonsäuremethylester | 88-89 |
| 34 | 3-Hydroxy-5-oxo-4-(4-trifluormethoxybenzoyl)-3-cyclohexencarbonsäuremethylester | 93-94 |
| 35 | 3-Hydroxy-5-oxo-4-(4-trifluormethoxybenzoyl)-3-cyclohexencarbonsäureethylester | 78-79 |

| Beispiel | Name der Verbindung | Physikalische Konstante Fp. ($^{o}$C) / $n_D^{20}$ |
|---|---|---|
| 36 | 4-(3,4-Dichlorbenzoyl)-3-hydroxy-5-oxo-3-cyclohexencarbonsäure-Dinatriumsalz | 292 |
| 37 | 4-(4-Difluormethoxybenzoyl)-3-hydroxy-5-oxo-3-cyclohexencarbonsäureethylester | 78-80 |
| 38 | 4-(4-Difluormethoxybenzoyl)-3-hydroxy-5-oxo-3-cyclohexencarbonsäuremethylester | 92-95 |
| 39 | 3-Hydroxy-4-(4-methylsulfonylbenzoyl)-5-oxo-3-cyclohexencarbonsäuremethylester | 137-139 |
| 40 | 3-Hydroxy-4-(4-methylsulfonylbenzoyl)-5-oxo-3-cyclohexencarbonsäureethylester | 105 |
| 41 | 3-Hydroxy-4-(4-methylthiobenzoyl)-5-oxo-3-cyclohexencarbonsäureethylester | 93-94 |
| 42 | 3-Hydroxy-4-(4-methylthiobenzoyl)-5-oxo-3-cyclohexencarbonsäuremethylester | 108-109 |
| 43 | 4-(4-Ethylthiobenzoyl)-3-hydroxy-5-oxo-3-cyclohexencarbonsäuremethylester | 69-71 |
| 44 | 4-(4-Ethylthiobenzoyl)-3-hydroxy-5-oxo-3-cyclohexencarbonsäureethylester | 70-71 |

Die folgenden Beispiele erläutern die Anwendungsmöglichkeiten der erfindungsgemäßen Verbindungen:

Beispiel A: **Wuchshemmwirkung bei Reis und Gerste**

Reis und Gerste wurden in humusreicher Erde bis zum 2-Blatt-Stadium angezogen. Danach erfolgte eine Spritzapplikation der Wirkstoffe in Aufwandmengen von 0,5 und 1,0 kg Wirkstoff/ha. 14 Tage nach der Behandlung wurde die Länge der Halme von der Bodenoberfläche bis zum Ansatz des obersten voll entwickelten Blattes gemessen. Die so erzielten Daten wurden in Relation zur Kontrolle gesetzt und die Wuchshemmwirkung nach folgendem Schema bewertet:

0 = ≥ 91 % der Wuchshöhe der unbehandelten Kontrolle

1 = 81 - 90 % der Wuchshöhe der unbehandelten Kontrolle

2 = 71 - 80 % der Wuchshöhe der unbehandelten Kontrolle

3 = 61 - 70 % der Wuchshöhe der unbehandelten Kontrolle

4 = ≤ 60 % der Wuchshöhe der unbehandelten Kontrolle

| Erfindungsgemäße Verbindungen | Aufwandmenge (kg/ha) | Wuchshemmwirkung Gerste | Reis |
|---|---|---|---|
| Beispiel 1 | 0,5 | 2 | 3 |
| | 1,0 | 3 | 4 |
| Beispiel 4 | 0,5 | 4 | 2 |
| | 1,0 | 4 | 2 |
| Beispiel 9 | 0,5 | 2 | 2 |
| | 1,0 | 4 | 4 |
| Beispiel 10 | 0,5 | 2 | 3 |
| | 1,0 | 2 | 3 |
| Beispiel 11 | 0,5 | 3 | 0 |
| | 1,0 | 4 | 2 |
| Beispiel 14 | 0,5 | 2 | 1 |
| | 1,0 | 2 | 3 |
| Beispiel 24 | 0,5 | 3 | 1 |
| | 1,0 | 4 | 1 |

| Erfindungsgemäße Verbindungen | Aufwandmenge (kg/ha) | Wuchshemmwirkung Gerste | Reis |
|---|---|---|---|
| Beispiel 33 | 0,5 | 3 | 1 |
| | 1,0 | 4 | 3 |
| Vergleichsmittel (gemäß EP-Anmeldung 0 137 963) | | | |
| 2-(2,4-Dichlorbenzoyl)-5,5-dimethyl-1-hydroxy-3-oxo-1-cyclohexen | 0,5 | 0 | 0 |
| | 1,0 | 0 | 0 |
| Vergleichsmittel (Handelsprodukt) | | | |
| Chlormequatchlorid | 0,8 | 2 | 1 |

Wie das Beispiel zeigt, sind die aus der gleichen Substanzklasse stammende bekannte Vergleichsverbindung und auch der kommerzielle Standard Chlormequatchlorid wesentlich schwächer wirksam als die erfindungsgemäßen Verbindungen.

Beispiel B: **Wuchshemmung bei Wasserreis**

Reispflanzen wurden in Plastiktöpfen bis zum 2- bis 3-Blattstadium angezogen und mit den erfindungsgemäßen Verbindungen behandelt. Die Applikation erfolgte in das Waser. 18 Tage nach der Behandlung wurde die Pflanzenhöhe bis zum Ansatz des obersten vollentwickelten Blattes gemessen. Die so ermittelten Daten wurden in Relation zur Kontrolle gesetzt und die Wuchshemmung nach folgendem Schema gewertet.

0 = > 90 % der Wuchshöhe der unbehandelten Kontrolle
1 = 81 - 90 % der Wuchshöhe der unbehandelten Kontrolle
2 = 71 - 80 % der Wuchshöhe der unbehandelten Kontrolle
3 = 61 - 70 % der Wuchshöhe der unbehandelten Kontrolle
4 = > 61 % der Wuchshöhe der unbehandelten Kontrolle

| Erfindungsgemäße Verbindungen | Aufwandmenge (kg/ha) | Wuchshemmung |
|---|---|---|
| Beispiel 3 | 1,0 | 1 |
| | 3,0 | 3 |
| Beispiel 8 | 1,0 | 3 |
| | 3,0 | 4 |
| Beispiel 9 | 1,0 | 2 |
| | 3,0 | 2 |
| Beispiel 10 | 1,0 | 3 |
| | 3,0 | 4 |
| Beispiel 13 | 1,0 | 2 |
| | 3,0 | 4 |
| Beispiel 14 | 1,0 | 3 |
| | 3,0 | 4 |
| Beispiel 24 | 1,0 | 2 |
| | 3,0 | 4 |
| Beispiel 27 | 1,0 | 2 |
| | 3,0 | 4 |
| Beispiel 28 | 1,0 | 3 |
| | 3,0 | 4 |

| Erfindungsgemäße Verbindungen | Aufwandmenge (kg/ha) | Wuchshemmung |
|---|---|---|
| Beispiel 30 | 1,0 | 2 |
| | 3,0 | 4 |
| Beispiel 33 | 1,0 | 3 |
| | 3,0 | 3 |
| Beispiel 38 | 1,0 | 4 |
| | 3,0 | 4 |

**Ansprüche**

1. 4-Benzoyl-3-hydroxy-5-oxo-3-cyclohexencarbonsäurederivate der allgemeinen Formel I

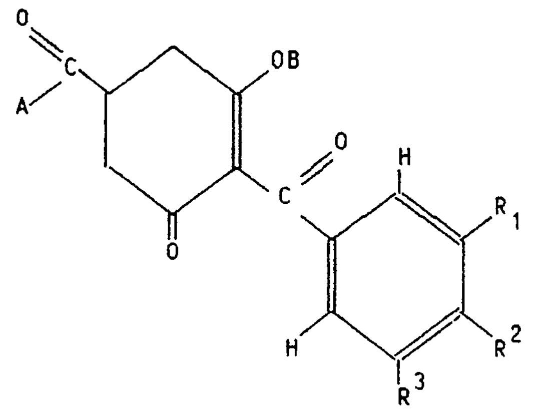

(I) ,

in der
A eine der Gruppen $OR^4$, $NR^5R^6$ oder OM,
B ein Wasserstoffatom oder ein Kation vom Typ M,
M ein Kation aus der Gruppe Lithium, Natrium und Kalium, ein Äquivalent aus der Gruppe Zink, Mangan, Calcium, Magnesium und Barium oder ein Ammoniumion der allgemeinen Formel

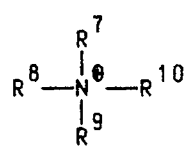

,

$R^1$, $R^2$ und $R^3$, unabhängig voneinander, ein Wasserstoffatom, ein Halogenatom, eine Hydroxy-, Cyano- oder Nitrogruppe, einen $C_1$-$C_6$-Alkyl-, $C_2$-$C_6$-Alkenyl-, $C_2$-$C_6$-Alkinyl-, $C_1$-$C_{10}$-Alkoxy-, Phenyl- oder Benzylrest, einen ein- oder mehrfach, gleich oder verschieden durch Halogen, Hydroxy, Halogen-$C_1$-$C_4$-alkyl, $C_1$-$C_4$-Alkoxy, Halogen-$C_1$-$C_4$-alkoxy, $C_1$-$C_4$-Alkylthio, $C_1$-$C_4$-Alkylsulfinyl oder $C_1$-$C_4$-Alkylsulfonyl substituierten $C_1$-$C_6$-Alkyl-, $C_2$-$C_6$-Alkenyl-, $C_2$-$C_6$-Alkinyl-, $C_1$-$C_{10}$-Alkoxy-, Phenyl- oder Benzylrest, einen $C_1$-$C_{10}$-Alkylthio-, $C_1$-$C_{10}$-Alkylsulfinyl- oder $C_1$-$C_4$-Alkylsulfonylrest oder eine der Gruppen $-(CH_2)_nCOA$, $-(CH_2)_nCOR^4$ oder $-NR^5R^6$, oder
$R^1$ und $R^2$ gemeinsam eine Methylendioxy-, Isopropylidendioxy- oder Ethylidendioxygruppe,
n eine Zahl 0, 1 oder 2,
$R^4$ ein Wasserstoffatom, einen $C_1$-$C_{18}$-Alkyl, $C_2$-$C_{12}$-Alkenyl- oder $C_2$-$C_{12}$-Alkinylrest, einen durch Halogen oder Cyano substituierten oder durch Sauerstoff oder Schwefel ein- oder mehrfach unterbrochenen $C_1$-$C_{18}$-Alkyl-, $C_2$-$C_{12}$-Alkenyl- oder $C_2$-$C_{12}$-Alkinylrest, einen $C_3$-$C_6$-Cycloalkyl- oder $C_3$-$C_6$-Cycloalkyl-$C_1$-$C_2$-alkylrest, einen durch Halogen oder Cyano substituierten $C_3$-$C_6$-Cycloalkyl- oder $C_3$-$C_6$-Cycloalkyl-$C_1$-$C_2$-alkylrest, einen Phenyl- oder Benzylrest, einen durch Halogen, Cyano, $C_1$-$C_4$-Alkyl, $C_1$-$C_4$-Alkoxy, $C_1$-$C_4$-Alkylthio, Nitro oder Trifluormethyl ein-oder mehrfach substituierten Phenyl- oder Benzylrest oder einen 5- oder 6-gliedrigen heterocyclischen Rest,
$R^5$ und $R^6$, die gleich oder verschieden sind, ein Wasserstoffatom, einen $C_1$-$C_{18}$-Alkyl-, $C_2$-$C_{12}$-Alkenyl- oder $C_2$-$C_{12}$-Alkinylrest, einen durch Halogen, Hydroxy oder Cyano substituierten oder durch Sauerstoff oder Schwefel ein- oder mehrfach unterbrochenen $C_1$-$C_{18}$-Alkyl-, $C_2$-$C_{12}$-Alkenyl-oder $C_2$-$C_{12}$-Alkinylrest, einen Phenyl- oder Benzylrest, einen ein- oder mehrfach durch $C_1$-$C_6$-Alkyl, Halogen, $C_1$-$C_6$-Alkoxy oder die Nitrogruppe substituierten Phenyl- oder Benzylrest oder
$R^5$ und $R^6$ gemeinsam mit dem benachbarten Stickstoffatom die Morpholino-, Piperidino- oder Pyrrolidinogruppe und

$R^7$, $R^8$, $R^9$ und $R^{10}$, die gleich oder verschieden sind, ein Wasserstoffatom, einen $C_1$-$C_6$-Alkyl-, Phenyl-, Benzyl- oder Phenylethylrest, einen durch Halogen, Hydroxy oder $C_1$-$C_6$-Alkoxy substituierten $C_1$-$C_6$-Alkyl-, Phenyl-, Benzyl- oder Phenylethylrest
bedeuten.

2. Verfahren zur Herstellung von 4-Benzoyl-3-hydroxy-5-oxo-3-cyclohexencarbonsäurederivaten der allgemeinen Formel I gemäß Anspruch 1, dadurch gekennzeichnet, daß man

A) eine Verbindung der allgemeinen Formel II

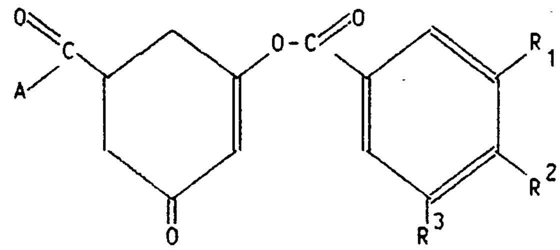

(II) ,

in der A, $R^1$, $R^2$ und $R^3$ die unter der allgemeinen Formel I genannten Bedeutungen haben, einer katalytisch geförderten Umlagerungsreaktion unterwirft, oder

B) eine Verbindung der allgemeinen Formel III

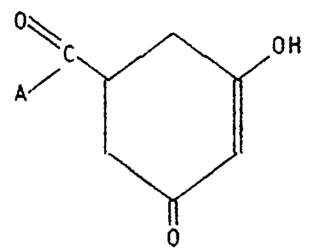

(III) ,

in der A die unter der allgemeinen Formel I genannte Bedeutung hat, in Gegenwart einer Lewis-Säure und einer geeigneten Base mit einem Benzoylcyanid der allgemeinen Formel IV umsetzt

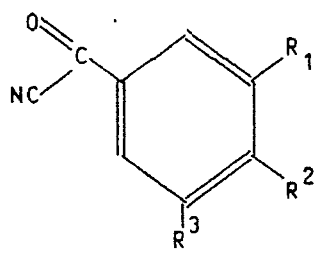

(IV) ,

in der $R^1$, $R^2$ und $R^3$ die unter der allgemeinen Formel I genannten Bedeutungen haben, oder

C) eine Verbindung der allgemeinen Formel I a

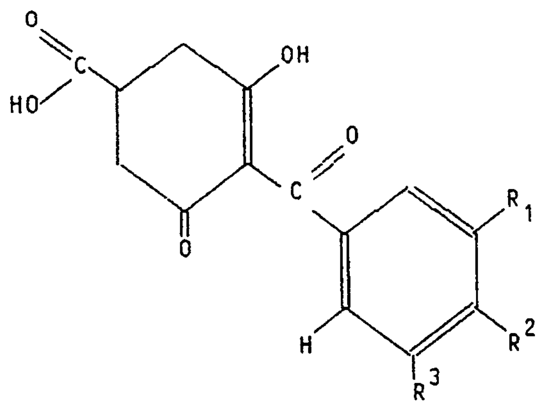

(I a) ,

in der R', $R^2$ und $R^3$ die unter der allgemeinen Formel I genannten Bedeutungen haben, mit Alkoholen oder Aminen der allgemeinen Formeln V oder VI

$R^4OH$ (V) $HNR^5R^6$ (VI),

in denen $R^4$, $R^5$ und $R^6$ die unter der allgemeinen Formel I genannten Bedeutungen haben, zur Reaktion bringt.

3. Mittel mit wachstumsregulierender Wirkung, gekennzeichnet durch einen Gehalt an mindestens einer Verbindung gemäß Anspruch 1.

4. Verwendung von Mitteln gemäß Anspruch 3 zur Wachstumsbeeinflussung von Kulturpflanzen.

5. Verfahren zur Herstellung von Mitteln gemäß Anspruch 3, dadurch gekennzeichnet, daß man eine oder mehrere Verbindungen gemäß Anspruch 1 mit Träger- und/oder sonstigen Hilfsstoffen vermischt.